Europäisches Patentamt

European Patent Office

Office européen des brevets

(11) Publication number: **0 152 298**
**A2**

# (12) EUROPEAN PATENT APPLICATION

(21) Application number: **85300885.2**

(22) Date of filing: **08.02.85**

(51) Int. Cl.⁴: **C 12 Q 1/22**
**A 61 L 2/26**

(30) Priority: **10.02.84 US 579159**

(43) Date of publication of application:
**21.08.85 Bulletin 85/34**

(84) Designated Contracting States:
**DE FR GB**

(71) Applicant: **AMERICAN HOSPITAL SUPPLY CORPORATION**
**One American Plaza**
**Evanston, IL 60201(US)**

(72) Inventor: **Bornhoeft, John W.**
**643 Hillside**
**Elmshurst, IL 60126(US)**

(72) Inventor: **Leal, Scott**
**5108 Sandberg Drive**
**McHenry, IL 60050(US)**

(74) Representative: **Knott, Stephen Gilbert et al,**
**MATHISEN, MACARA & CO. The Coach House 6-8**
**Swakeleys Road**
**Ickenham Uxbridge UB10 8BZ(GB)**

(54) **Apparatus and method for dertermining the effectiveness of sterilization.**

(57) A test container comprises a vial (11) within which is disposed a sealed media-containing ampoule (12) and a test strip (14) having a predetermined form of micro-organism thereon. A closure member (15) is threadedly engageable with the vial for movement between a first position, which permits entry of sterilizing atmosphere into the vial and into contact with micro-organisms on the test strip, and a second position (not shown) which simultaneously causes fracturing the ampoule (12) and seals (at 36) the interior of the vial (11) so that gas generated by viable micro-organisms in contact with the media during incubation are contained in the vial. By analyzing the gas generated by the surviving micro-organisms a rapid, quantitative indication is given of the effectiveness of the sterilizing procedure.

Fig. 3.

APPARATUS AND METHOD FOR DETERMINING THE EFFECTIVENESS

OF STERILIZATION

The present invention relates generally to a method and apparatus for determining the effectiveness of sterilization procedures, and more particularly, to an improved method and apparatus for determining the presence or absence of live microorganisms following their passage through a sterilization cycle.

Numerous medical items used in hospitals, such as sutures, catheters, gloves, bandages, and the like must be sterilized prior to use, either by the manufacturer or the hospital. Sterilizing media such as steam, dry heat, sterilizing gases and irradiation have been employed for such purpose. Problems have arisen, however, in determining the completeness or efficacy of the sterilization procedure, and the need has long existed for means whereby the effectiveness of a sterilization cycle may be determined quickly and reliably.

Heretofore, one method of testing the effectiveness of sterilization has involved the use of a small vial which contains a spore strip (i.e., generally a piece of absorbent paper having a predetermined form of microbial spores thereon) and a sealed ampule of a sterile nutrient media. The vial typically has means, such as a semipermeable membrane or passages in the closure, that permits steam or gas to enter the vial during the sterilization cycle. The vial is placed in the sterilization chamber along with the items to be sterilized so that the spores within the vial are exposed to the sterilization along with, and in the same manner, as the items to be sterilized. Upon completion of the sterilization cycle, the vial is removed, and the inner ampule

- 2 -

0152298

broken to release the media to mix with any spores that may have survived the sterilization. The vial is then incubated for a period of one to several days, depending on the strain of spores used. If viable spores remain, their germination and growth, utilizing the nutrient of the media, will alter the pH of the solution, causing a visible change in color of the solution or an increase in turbidity, thereby indicating the lack of complete sterilization. The foregoing sterilization detection procedure, however, suffers from various drawbacks, including difficulties in breaking of the media containing ampule without contaminating the media, the relatively long period of time required before results can be determined, the ambiguity in the results that sometimes are obtained, and the inability to make reliable quantitative determinations of the degree of sterilization.

While systems have been available for more quickly determining biological activity, such systems, heretofore, have not been viewed as being adaptable for use in sterilization testing procedures. For example, in effecting bacterial determinations in samples of blood, spinal fluid, or the like, it is known to inoculate a test sample containing or believed to contain bacteria or microorganisms into a sealed vial containing a radioisotope labeled nutrient media. The sealed vial is then incubated so that any microorganisms present in the sample will metabolize, oxidize, or otherwise process the nutrient media and evolve or respirate a gaseous carbon dioxide which includes the radioisotope. After a relatively short incubation period, on the order of four to eight hours, the vial can be removed from the incubating chamber and utilized in a known radioactive gas detection apparatus, which quantitatively measures the evolved gas and the rate of evolution to provide an indication of the presence of and the degree of activity of micro-organisms in the test sample. Because such radio-activity media containing vials are typically maintained in a gas-tight sealed condition throughout the procedure, they have not been utilized in sterilization indicator procedures where the sterilizing atmosphere must enter the vial.

The invention consists in a test container for use in determining the effectiveness of sterilization comprising: a vial; and characterised by means for supporting within said vial a test strip having a predetermined form of micro-organism thereon, and an aqueous growth medium, such that said test strip is normally maintained out of contact with said medium, a closure member mountable in a first position on said vial which permits entry of a sterilizing atmosphere into said vial and into contact with the micro-organisms on said test strip during a sterilization cycle, and means for permitting said test strip to be brought into contact with said aqueous medium in said vial at a selected time after the sterilizing cycle; said closure member being movable to a second position on said vial which seals the interior of said vial so that gases generated by viable micro-organisms in contact with said medium during incubation thereof are contained in said vial to permit their analysis.

According to a further aspect, the invention consists in a method of testing the effectiveness of sterilization comprising the steps of: placing with items to be sterilized a vial containing a test strip with a predetermined form of micro-organisms thereon and an aqueous growth medium such that when brought in contact with viable micro-organisms on said test strip and incubated for a predetermined period will result in the evolution of detectable gas, maintaining the test strip in said vial out of contact with the said medium therein while exposing the test strip to the sterilizing atmosphere during a sterilization cycle along with the items to be sterilized, contacting the test strip with the medium in said vial following the sterilization cycle, sealing the medium and test strip in the vial, incubating the vial and its sealed contents for a predetermined period to cause any viable micro-organisms remaining following the sterilization cycle to evolve a gas, and analyzing the gas evolved in said sealed vial to determine the effectiveness of the sterilization cycle.

The invention provides quick and reliable quantitative testing of the effectiveness of any suitable sterilizing process.

In order that the invention may be better understood, several preferred embodiments of the invention will now be described, by way of example only, with reference to the accompanying schematic drawings, wherein:

Figure 1 is a side elevational view of a sterilizing test container embodying the present invention and for use with the method of the present invention;

Figure 2 is an exploded perspective of the test container shown in Figure 1;

Figure 3 is an enlarged vertical section of the container shown in Figure 1, taken in the plane of line 3-3, showing the closure member thereof in a first locked position which permits entry of a sterilizing atmosphere into the interior of the container;

Figure 4 is a vertical section, similar to Figure 3, but showing the closure thereof in a second locked position sealing the interior of the container in gas-tight relation to the outside atmosphere;

Figures 5 and 6 are horizontal sections taken in the planes of lines 5-5 and 6-6, respectively in Figure 3;

Figure 7 is a horizontal section taken in the plane of line 7-7 in Figure 4;

Figure 8 is a perspective of a radio-active gas detecting apparatus in operative engagement with a container such as shown in Figure 1;

Figure 9 is a representation of the detecting apparatus shown in Figure 8;

Figure 10 is a vertical section of an alternative embodiment of test container according to the present invention;

Figure 11 is a horizontal section taken in the plane of line 11-11 in Figure 10;

Figure 12 is a verticval section of another alternative embodiment of a test container according to the present invention;

Figure 13 is a vertical section of still another alternative embodiment of test container, showing the closure member thereof in a vented position;

Figure 14 is a vertical section of the test container shown in Figure 13, but showing the closure member thereof in a vial sealing condition; and

Figure 15 is a vertical section of still a further alternative embodiment of test container.

While the invention is susceptible of various modifications and alternative constructions, certain preferred embodiments have been shown in the drawings and will be described below in detail. It should be understood, however, that there i no intention to limit the invention to the specific forms described but, on the contrary, the intention is to cover all modifications, alternative constructions and equivalents falling within the scope of the invention.

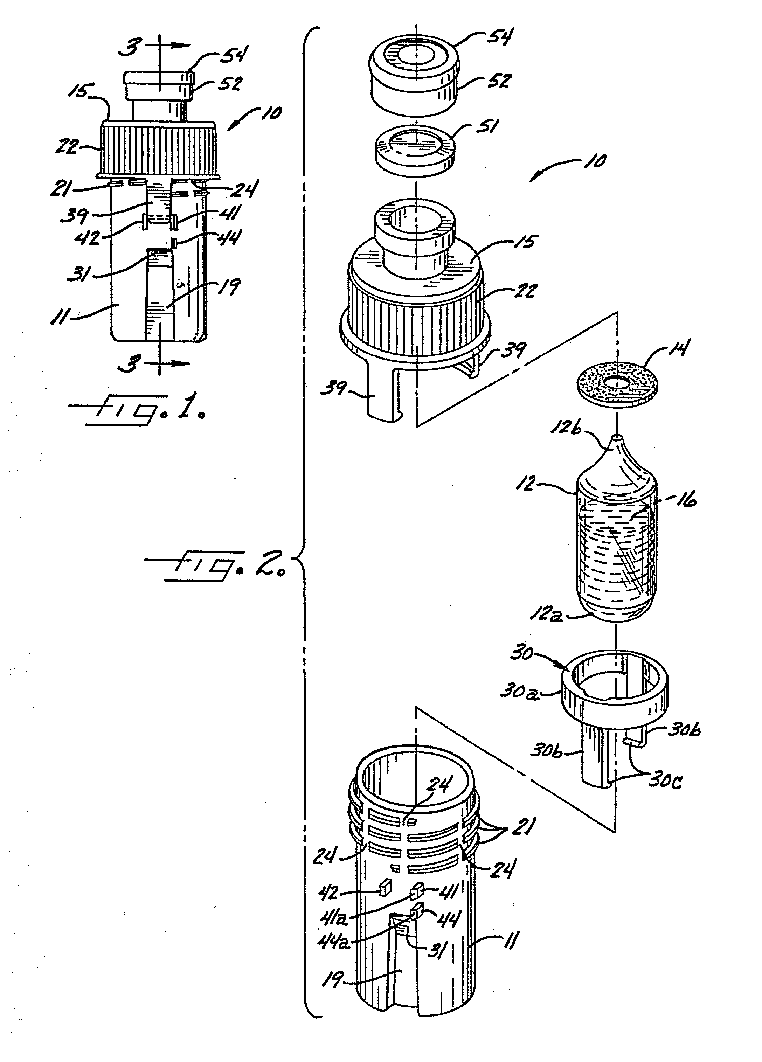

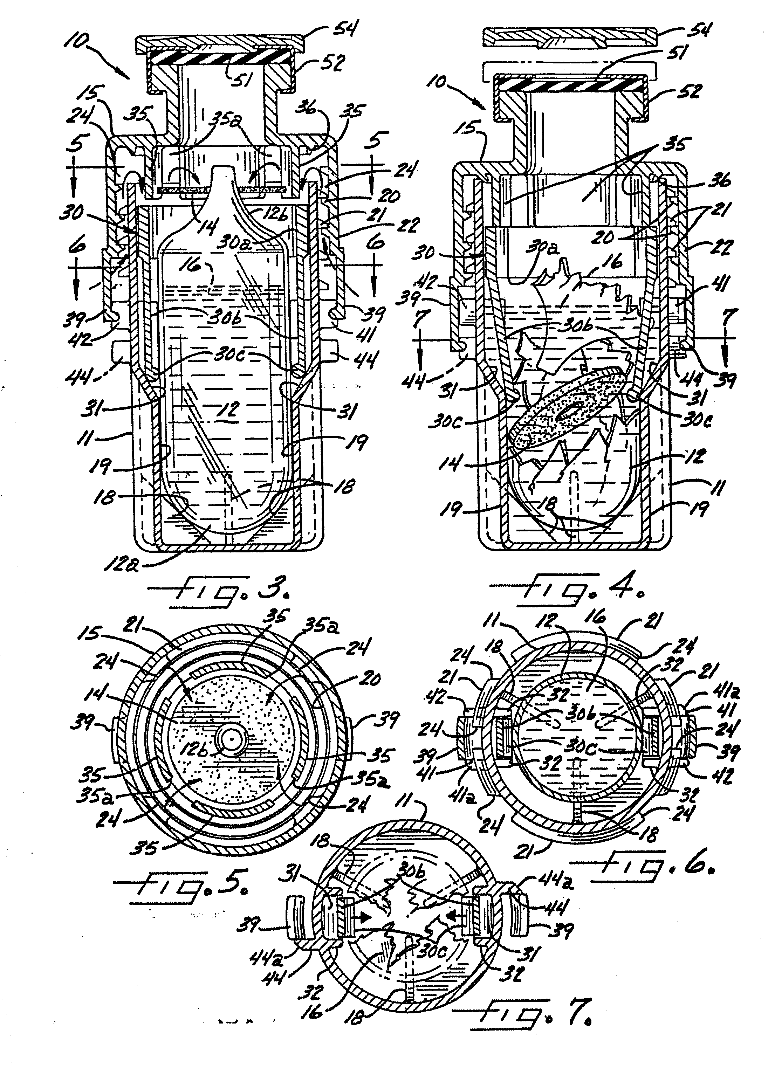

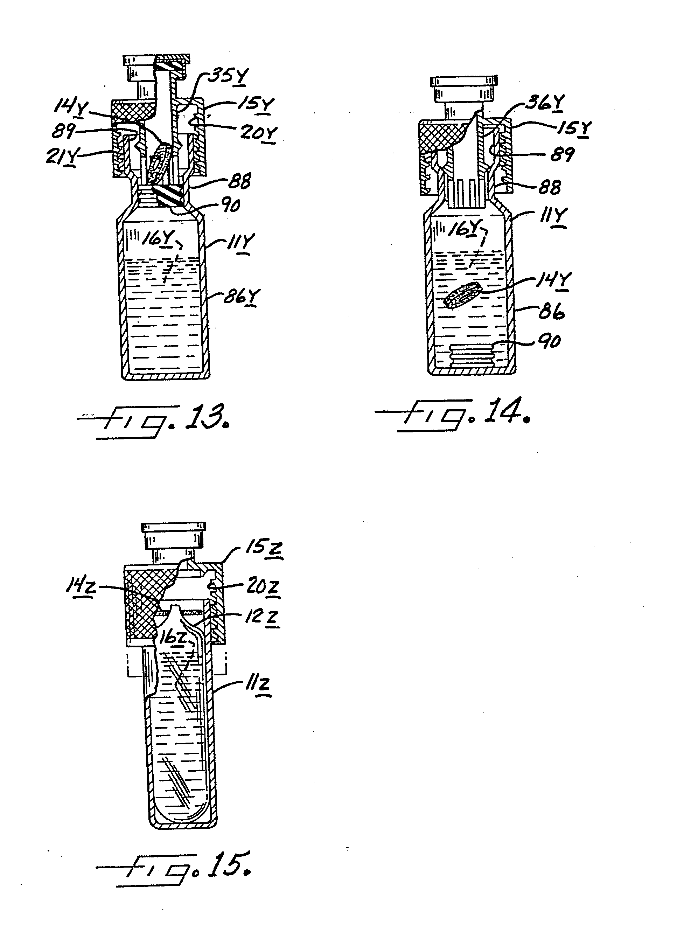

Referring more particularly to FIGS. 1-7 of the drawings, there is shown an illustrative test container 10 adapted for use in testing the effectiveness of sterilization procedures in accordance with the present invention. The container 10 includes an outer vial 11 that is closed at the bottom end, a sealed media containing ampule 12 disposed in upstanding relation within the vial 11, an annular disc-shaped test strip 14 positioned on the upper end of the ampule 12, and a closure member 15 positionable onto the upper open end of the vial 11. The illustrated vial 11 has a generally cylindrical configuration and preferably is made of a transparent, relatively rigid plastic material.

The ampule 12 may be of a conventional configuration, in this instance having a generally cylindrical shaped central portion with a spherical bottom end 12a and a narrowed upwardly extended top end 12b. The ampule 12 preferably is made of a frangible glass material and contains in hermetically sealed condition a predetermined volume of an aqueous nutrient media 16. For supporting the ampule 12 concentrically within the vial 11, the vial is formed with a plurality of ribs 18 extending upwardly and outwardly from a central location in the bottom of the vial. A pair of inwardly recessed, channel-shaped ribs 19 are formed in opposed sides of a lower portion of the vial for supporting the sides of the ampule 12 in an upright position.

The test strip 14 may be a strip of absorbent material of a known type bearing a predetermined form of microorganisms for the sterilization procedure to be tested, preferably in the form of bacterial spores. As is known in the art, the spore bearing microorganism Bacillus stearotheromophilus may be utilized when detecting the effectiveness of steam sterilization. Bacillus substilis commonly is employed in testing the effectiveness of ethylene oxide and dry heat sterilization cycles, and Bacillus pumilus is used when verifying irradiation sterilization cycles. It will be understood that combinations of such microorganisms may be utilized on a single spore strip. While reference may be made herein to spores as the test microorganisms, it will be understood that microorganisms other than spore formers may be used in conjunction with the test strips.

For securing the closure member 15 on the end of the vial 11, the closure member 15 is formed with internal threads 20 which are adapted for threadable engagement with external threads 21 formed on the upper portion of the vial 11. To facilitate turning of the closure member 15 relative to the vial 11, and thus advancement of the closure member onto the vial, the closure member in this instance is formed with a knurled outer peripheral gripping surface 22.

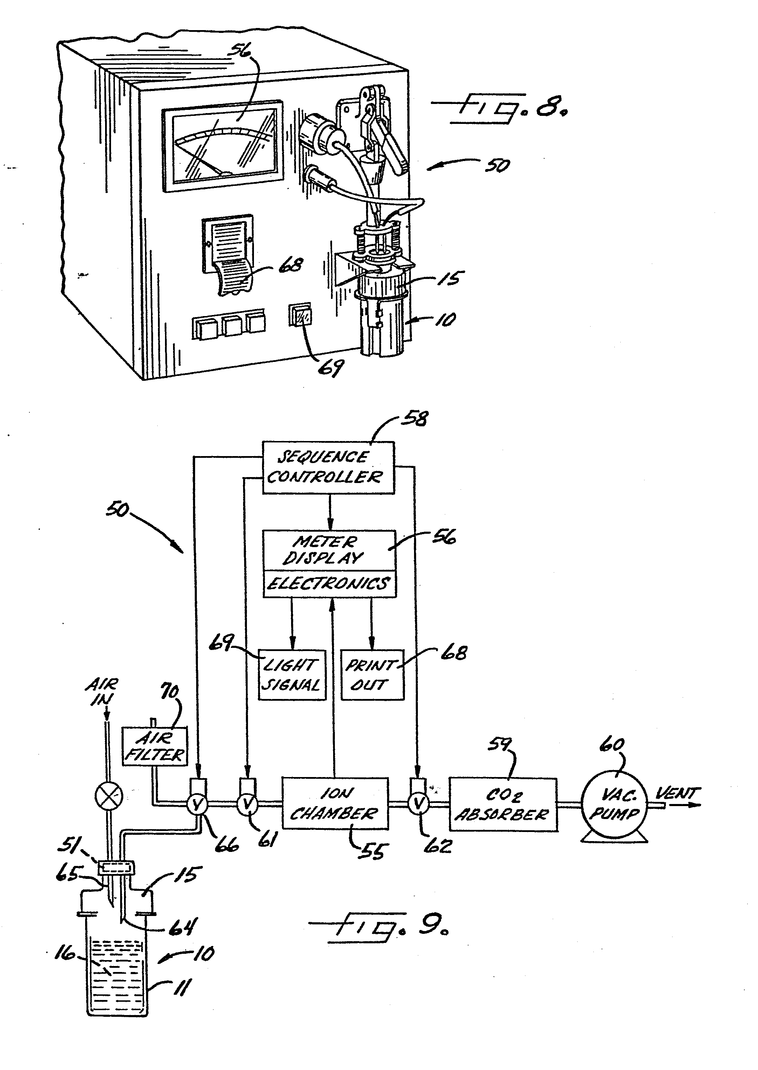

In one embodiment of the invention, the closure member and vial threads define a gas venting passageway, and the closure member is positionable between a first predetermined locked position that permits a sterilizing atmosphere to pass through the passageway into the interior of the vial and a second predetermined locked position which simultaneously seals the interior of the container and effects the breakage of the ampule to release the media. To this end, in the illustrated embodiment the threads 21 of the vial 11 are formed with a plurality of longitudinally aligned recesses or vents 24 at circumferentially spaced locations about the outer periphery of the vial. Since the threads 20 of the closure member 15 engage the vial threads 21 in
outwardly spaced relation to the outer perimeter of
the vial, and since the vents 24 in the vial threads
are recessed flush with the outer perimeter of the
wall, it can be seen that with the closure member in
threaded engagement on the end of the vial, as shown
in FIG. 3, the vents define a plurality of
longitudinal passageways which permit sterilizing
atmosphere from outside the container to pass
upwardly from under the closure member, through the
vent passageways, and into the interior of the vial.

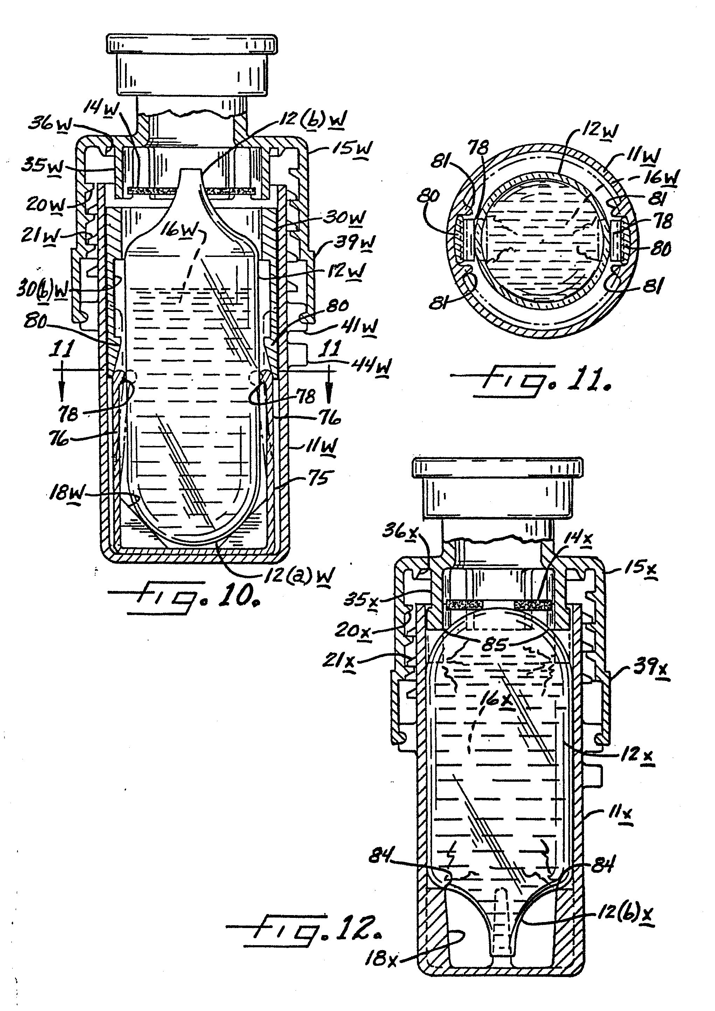

In response to
further threaded engagement of the closure member
onto the vial, means are provided for simultaneously
fracturing the ampule contained therein and sealing
the interior of the vial. For this purpose, a ram 30
is disposed in the vial concentrically about the
ampule 12. The ram 30 in this instance comprises an
annular collar portion 30a disposed concentrically
about the upper portion of the ampule 12 and a pair
of pincher arms 30b extending in depending fashion
from collar 30a on opposed sides of the ampule, the
pincher arms 30b being located in an annular space
defined between the ampule and the peripheral wall of
the vial, as shown in FIG. 3. The ram 30 preferably
is made of a plastic material which permits some
degree of flexibility of the pincher arms 30b, as
will become apparent. For supporting the ram 30 in a
raised, normally stored condition, the upper ends of
the recessed ribs 19 are formed with upwardly and
outwardly tapered ramps 31 which extend from a
location in close relation with the ampule 12 to the
peripheral wall of the vial 11. The ramps 31 are
disposed on opposed sides of the vial, with lower
most ends of the ram pincher arms 30b supported thereon. For retaining the pincher arms 30b on the ramps 31 in proper circumferentially spaced relation to the ampule 12, the peripheral wall of the vial is formed with pairs of ribs 32 within which the pincher arms 30b are retained for relative vertical movement (FIGS. 5 and 6). To facilitate movement of the pincher arms 30b along the ramps, as will become apparent, the pincher arms have lower most rounded ends 30c.

For moving the ram 30 from the raised, stored position, shown in FIG. 3, to an ampule breaking position, shown in FIG. 4, the closure member 15 is formed with a depending, concentrically-disposed, annular bearing member 35 which upon advancement of the closure member 15 into further threaded engagement with the vial 11 by relative rotation thereof, the bearing member 35 is brought into engagement with an upper end of the ram and forces it in a downward direction. Such downward movement of the ram 30 causes the pincher arms 30b to be cammed downwardly and inwardly by the ramps 31 to a position which ultimately fractures the ampule 12, thereby releasing the media 16 and allowing the test strip 14 to fall into contact with the media.

In order to seal the interior of the vial 11 upon breakage of the ampule 12, the closure member 15 may be threaded tightly onto the vial until the upper end of the vial engages an annular sealing rib 36 formed on the underside of the closure member. The sealing rib 36 preferably is formed with the same flexibility and is directed inwardly at an angle to the longitudinal axis of the closure member such that when the upper end of the vial engages the side of the sealing rib 36 (as shown in FIG. 4), a gas-tight seal is effected about the entire upper end of the vial.

To facilitate reliable selective locating of the closure member 15 in a vented condition (FIG. 3) or in a sealing condition (FIG. 4), means are provided for positively establishing such positions in a manner that permits the user of the container to see, feel, and hear when the closure member is properly located. To this end, the closure member 15 is formed with a pair of depending locking legs 39 on opposed sides thereof and the vial 11 is formed with pairs of outwardly extending lugs 41, 42 on opposed sides, the lugs 41, 42 defining recesses for positively receiving and holding the respective closure member locking legs in the first predetermined or venting condition, as shown in FIGS. 3 and 6. To facilitate positioning of the closure member locking legs in such first locked position as an incident to rotation of the said closure member onto the end of the vial, the locking legs preferably have limited flexibility and the first lug 41 of each pair is formed with a chamfered side 41a that permits the respective locking leg to be cammed up over the lug 41 and then snapped into the recess. The closure member locking legs 39 are thereby positively retained in the recesses, maintaining the closure member 15 in predetermined desired orientation on the vial for enabling reliable passage of a sterilizing atmosphere through the vented passageway in the vial threads and into the interior of the container. To further facilitate entry of the sterilizing atmosphere into the container, the annular bearing member 35 of the closure member 15 is formed with circumferentially spaced openings 35a which are aligned with the vents 24 in the vial threads when the closure member is locked in such vented position (FIG. 5).

To locate the closure member 15 in the sealed condition (FIG. 4), the closure member may be rotated such that the legs are forced over the second lugs 42 of each pair so as to free the closure member for further rotation and threaded engagement onto the vial. As the closure member 15 approaches the sealed condition, shown in FIG. 4, the closure member locking legs 39 will engage second position lugs 44, in this case located on diametrically opposed sides of the vial 11 in spaced axial relation below the lugs 41 of the first set. To facilitate movement of the locking legs 39 into this locked position, the lugs 44 again are formed with a forward chamfered surface 44a over which the legs 39 are cammed and then snapped into locked position behind the lugs 44 which positively retain the closure member in the sealed condition. It will be appreciated that the locking legs 39 and lugs 41, 42, 44 enable the user of the container 10 to easily establish the proper venting position of the enclosure member 15, as well as the sealed position, by visually observing the positioning of the locking legs, as well as by hearing and feeling when the closure member is properly located.

From the foregoing, it can be seen that with the closure member 15 locked in a first or venting position (FIG. 3), the test container 10 may be placed in a sterilization chamber along with items to be sterilized so that the microorganisms on the test strip 14 within the vial 11 are exposed to the sterilization cycle along with, and in the same manner, as the items being sterilized. Upon completion of the sterilization cycle, the test container 10 can be removed and the inner ampule 12 easily broken by simply turning the closure member 15 into further threaded engagement with the vial 11, which will automatically force the ram 30 downwardly into the ampule, breaking the ampule to release the media 16 and allowing the test strip 14 to fall into and mix with the media (FIG. 4). As an incident to such further threaded engagement of the closure member 15 on the vial 11, the upper end of the vial will be moved into engagement with the sealing ribs 36 of the closure member, sealing the contents of the test container 10 from the outside environment with the closure member legs being forced over the lugs 44 and snapped into locked position, positively retaining the closure member in such sealed condition.

In keeping with the invention, the aqueous media 16 contained in the ampule 12 is a sterile solution of a bacterial growth nutrient which contains a component or components having a radioactive isotope such that when brought in contact with viable microorganisms on the test strip 14 and incubated for a determined period will result in the evolution of detectable radioactive carbon dioxide gas. Preferably the radioactive component is a sugar, such as glucose, in which all of the carbons are the 14C radioactive isotope. It will be understood by one skilled in the art that other radioactive labeled bacterial growth nutrient medias could be used. Because the closure member 15 is brought into sealing relation with the vial 11 as an incident to fracturing of the media ampule 12, gases generated within the test container during subsequent incubation of the contents of the container are retained therein. Typically, incubation periods from two to several hours are sufficient to cause the evolution of detectable radioactive gases from viable microorganisms.

Following incubation of the sealed test container, the container is utilized in a radioactive gas analysis apparatus 50 that is adapted to remove from the container 10 gases generated during incubation, analyze the gases, and make a quantitative determination as to the degree of biological activity that survived the sterilization. To facilitate removal of the gas from the test container 10 by the analysis apparatus 50, the closure member 15 has an upper end formed from a rubber septum 51, in this case being mounted at the top of the closure member 15 and retained in position by an annular closure ring 52. The septum 51 is made of a suitable rubber material which effectively maintains the gas-tight integrity of the closure member 15, while permitting insertion therethrough of appropriate hypodermic needles to enable the withdrawal of accumulated gases from the interior of the container without entry of the outside environment. To maintain the septum 51 in a clean condition prior to use, a removable end cap 54 is fixed on the top of the closure member 52, as shown in FIG. 3, and can be removed from the container, as shown in FIG. 4.

For removing and analyzing gases in the sealed container, the radioactive gas detecting apparatus 50, shown in FIGS. 8 and 9, may be of a known type, such as Bactec models 301 or 460 sold by Johnson Laboratories, Inc. and described in U.S. Patent No. 3,935,073. Such apparatus includes an ion chamber 55, and electron meter and meter display 56, a sequence controller 58, a $CO_2$ absorber 59, a vacuum pump 60, and control valves 61 and 62. A test container 10 may be brought into position such that sterile hypodermic needles 64 and 65 can be caused to penetrate the rubber septum 51 of the test container
closure member 15 so that gas is drawn from the
container through needle 64 and valve 66 and into ion
chamber 55 for measurement of radioactivity.  The
amount of activity, in this instance, is displayed on
the display meter 56, as well as on a printout 68.
It will be understood that by appropriate
electronics, in the event the measurement exceeds a
predetermined level, a light 69 may be actuated.  The
amount of measured radioactivity, thereby, will
reflect the existence of viable microorganism
remaining in the test container following
sterilization, as well as quantitatively indicate the
extent such sterilization was effective.

After the gaseous atmosphere in the container
has been sampled and analyzed, the valve 66 can be
actuated to connect the ion chamber 55 with filtered
air 70 to allow the ion chamber 55 to be flushed with
clean air so that the apparatus is in condition to
analyze the next sample.  In practice, it has been
found that determinations as to the effectiveness of
the sterilization cycle can be made by the apparatus
55 in a matter of minutes following completion of
incubation, which typically takes only between about
two to several hours.

Referring now to FIGS. 10 and 11, there is shown
an alternative design of the test container embodying
the present invention, wherein items similar to those
described above have been given similar reference
numerals with the distinguishing suffix "w" added.
The test container 10w includes a closure member 15w,
test strip 14w, and a radioisotope media containing
ampule 12w, all identical to those described above.
The vial 11w in this instance has a uniform,
cylindrical configuration, again having a closed bottom with ampule supporting ribs 18w and an externally threaded upper end. Hence, with the closure member 15w in a first locked position, as shown in FIG. 10, the test strip 14w within the test container may be exposed to a sterilization cycle.

To effect breakage of the ampule 12w upon threaded advancement of the closure member 15w onto the vial 11w following a sterilization cycle, the lower half of the ampule 12w in this case is supported within a generally U-shaped pincher 75 having upstanding pincher arms 76 disposed on diametrically opposed sides of the ampule. The pincher arms 76 are formed with ampule engaging beads 78 at their upper terminal ends. For moving the pincher arms 76 into ampule breaking engagement, a ram 30w is concentrically disposed about the upper half of the ampule 12w and is formed with depending legs 30(b)w having wedge-shaped terminal ends 80. The ram legs 30(b)w in this case are located in closely adjacent relation to the peripheral wall of the vial 11w. For maintaining the pincher arms 76 and ram legs 30(b)w in proper relation on opposite sides of the ampule, the inside wall of the vial 11w is formed with pairs of inwardly extending ribs 81 which define recesses within which the pincher arms and ram legs are disposed. Upon threaded advancement of the closure member 15w onto the vial 11w as described previously, a bearing member 35w of the closure member 15w will engage the ram 30w and force it in a downward direction such that the wedge-shaped terminal ends 80 are forced between the pincher arms 76 and vial wall, thereby forcing the beaded terminal ends 78 of the pincher arms 76 inwardly against the ampule 12w. The inward forces are concentrated at a central, relatively vulnerable area of the ampule, so as to cause reliable fracturing of the ampule. As an incident to fracturing of the ampule 12w, the closure member 15w is moved into sealing relationship with the upper end of the vial 11w as previously described, enabling the test container 10w to then be processed through incubation and radioactive gas detection process steps to quantitatively determine the effectiveness of the cycle sterilization in which the test container was used.

Referring now to FIG. 12, another alternative embodiment of test container is shown, wherein items similar to those described above have been given similar reference numerals with the distinguishing suffix "x" added. The test container 10x includes a closure member 15x similar to that previously described, a generally cylindrical shaped vial 11x, and a radioactive isotope labeled media containing ampule 12x again similar to that previously described but disposed in inverted fashion in the vial with the neck portion 12(b)x thereof oriented downwardly. The test strip 14x in this case is supported on the spherically-shaped upper portion of the ampule 12x and is confined within the bearing member 35b of the closure member. The underside of the ampule 12x is supported at points adjacent the neck 12(b)x by relatively sharp corners 84 of a plurality of rectangular-shaped ribs 18x integrally formed in the bottom of the vial 11x. The bearing member 35x of the closure member 15x in this instance is formed with an enlarged lower end having a relatively sharp annular edge 85 that is engageable with the upper end of the ampule 12x.

With the closure member 15x in the position illustrated in FIG. 12, sterilizing atmosphere can enter the vial in the manner previously described during a sterilization cycle. Upon completion of a sterilization cycle, the closure member 15x can be threadedly advanced onto the vial 11x causing the bearing member 35x to engage and axially compress the ampule, 11x, with high stress concentrations occurring at the points of contact between the opposed ends of the bearing member 35x and the vial ribs 18x. The application of such concentrated forces on the ampule 12x again will cause reliable breakage of the ampule as an incident to moving the closure member 15x into sealing engagement on the end of the vial 11x.

Referring now to FIGS. 13 and 14, there is shown still another embodiment of test container, wherein items similar to those described above have been given similar reference numerals with the distinguishing suffix "y" added. The test container 10y includes a vial 11y having a cylindrical body portion 86 which directly contains a quantity of aqueous radioactive isotope labeled media 16y, a reduced diameter neck portion 88, and an enlarged externally threaded upper end 89 with vented threads for receiving an internally threaded closure member 15y in a manner similar to that previously described. For isolating the media 16y from a test strip 14y and the outside environment during a sterilization cycle, a rubber stopper 90 is disposed in the neck portion 88, and the test strip 14y located on top of the stopper 90.

For simultaneously sealing the vial from the outside environment following sterilization and for enabling the test strip 14y to become immersed in the aqueous media 16y, the closure member 15y includes a depending annular-shaped bearing member 35y, similar to that described above but somewhat longer in length, which as an incident to threaded engagement of the closure member 15y from the vented position, shown in FIG. 13, to the vial sealing condition, shown in FIG. 14, is moved into engagement with and forces the rubber stopper 90 through the reduced neck portion 88 of the vial 14y, causing the stopper 90 and test strip 14y both to fall into the media 16y, as shown in FIG. 14. The test container 10y may then be incubated and utilized in radioactive gas detection apparatus as previously described.

Referring now to FIG. 15, still another test container is illustrated, wherein items similar to those described above have been given similar reference numerals with the distinguishing suffix "z" added. Test container 10z includes a cylindrical-shaped vial 11z, in this instance made of sufficiently flexible material to permit manual manipulation thereof. The vial 11z has an upper end formed with vented threads 21z which receive an internally threaded closure member 15z similar to that described above. Contained within the vial is a radioactive media containing ampule 12z having a test strip 14z supported on an upper neck portion thereof. With the closure member 15z in a vented position, as shown in FIG. 15, sterilizing atmosphere is permitted to pass into the interior of the vial 11z and into contact with the test strip 14z. Upon completion of the sterilization cycle, the closure member 15z may be threadedly advanced into sealing engagement with the end of the vial 11z, and the ampule 12z may be broken by manually manipulating the flexible body portion of the vial 11z, thereby causing the test strip to be dropped into contact with the aqueous media 16z. The test container may then be processed through incubation and the gas analysis steps as previous described.

From the foregoing, therefore, it can be seen that the method and apparatus of the present invention are adapted for quickly and reliably determining the effectiveness of sterilization procedures, which heretofore has taken between one and several days. Moreover, the method and apparatus are adapted to provide quantitative determinations as to the degree of sterilization. The test container of the present invention, furthermore, is easy and reliable to use during sterilization, incubation, and gas analysis, and may be employed in connection with steam, dry heat, gas, and irradiation sterilization procedures.

## CLAIMS

1.      A test container for use in determining the effectiveness of sterilization comprising: a vial (11); and characterised by means (12;90) for supporting within said vial (11) a test strip (14), having a predetermined form of micro-organism thereon, and an aqueous growth medium (16), such that said test strip (14) is normally maintained out of contact with said medium (16), a closure member (15) mountable in a first position on said vial (11) which permits entry of a sterilizing atmosphere into said vial (11) and into contact with the micro-organisms on said test strip (14) during a sterilization cycle, and means for permitting said test strip (14) to be brought into contact with said aqueous medium (16) in said vial (11) at a selected time after the sterilizing cycle; said closure member (15) being movable to a second position on said vial (11) which seals the interior of said vial (11) so that gases generated by viable micro-organisms in contact with said medium during incubation thereof are contained in said vial (11) to permit their analysis.

2.      A test container according to claim 1, in which the said supporting means comprises a sealed ampoule

0152298

(12) containing said aqueous medium (16) and made of frangible material, the means for permitting contact of the test strip (14) with the medium (16) comprises means (30) for fracturing said ampoule (12) in response to movement of said closure member (15) from said first position to said second position.

3. A test container according to claim 2, in which said closure member (15) is threadably engageable with the end of said vial (11) for threaded advancement between said first and second positions in response to rotation of said closure member (15) relative to the vial (11).

4. A test container according to claim 2 or 3, including first locking means (41,42) for positively locking said closure member (15) in said first position, and second locking means (44) for positively locking said closure member in said second, sealing position.

5. A test container according to any of claims 2 to 4, in which the said ampoule (12) contains an aqueous growth medium which includes a radio-active isotope component such that when brought in contact with viable micro-organisms on said test strip (14) and incubated for a determined period will result in the evolution of detectable radio-active carbon dioxide gas.

6.      A test container according to any of claims 2 to 5,  including means responsive to threaded advancement of  said  closure member (15) onto said vial  (11)  for breaking said ampoule (12).

7.      A test container according to claim 6, including ram  means  (30)  disposed within said  vial  (11)  for relative  longitudinal  movement,  said closure  member (15)  being  engageable with said ram means  (30)  upon threaded  advancement  onto  said  vial  (11)  for longitudinally moving said ram means to a position that causes breakage of said ampoule (12).

8.      A test container according to claim 7,  in which said  vial (11) is formed with downwardly and  inwardly tapered  ramps  (31) which support said ram means  (30) when said closure member (15) is in said first position and  which  cam said ram  means  into  ampoule-breaking position  upon  threaded  advancement of  said  closure member (15) on said vial (11) to said second position.

9.      A test container according to claim 8,  in which said ram means (30) includes a collar (30a) disposed in concentric relation about the ampoule (12) and a pair of flexible pincher legs (30b) depending from said collar in adjacent relation to said ampoule (12).

10.    A test container according to claim 3, or to any claim dependent on claim 3, in which the threads of said closure member (15) and vial (11) define a gas passageway between said closure member and vial when said closure member is in said first position.

11.    A test container according to claim 3 or to any claim dependent on claim 3, in which some of the threads of said vial (11) and closure member (15) are recessed to permit passage of gas between said closure member and vial when said closure member is in said first position.

12.    A test container according to claim 7, including pincher means (75) located in the bottom of said vial (11) and having a pair of upstanding pincher arms (76) disposed adjacent said ampoule (12), said ram means (30) including a pair of depending legs (30bw), and said ram means being movable in said vial in response to threaded advancement of said closure member (15) onto said vial so that said ram legs wedgedly force said pincher arms into ampoule-breaking engagement.

13.    A test container according to claim 3,  in which said closure member (15) includes bearing means (85) which, in response to threaded advancement of said closure member (15) onto said vial, axially compresses said ampoule (12) to effect breakage thereof.

14.    A test container according to claim 2 or 3, in which said vial (11) is made of flexible plastic material which can be manually manipulated to effect selected breakage of the ampoule (12,Fig.15) therein.

15.    A test container according to claim 1, in which said vial (11) is formed with an upstanding relatively narrow upper neck portion (88), and said test strip (14y) is annular shaped and disposed on said neck portion.

16.    A test container according to any preceding claim, in which said closure member (15) includes an upper end (52) closed by a rubber septum (51) which permits passage of gas-removing needles into the container (10) without entry of the outside environment.

17.    A test container according to any preceding claim, in which said closure member (15) is formed with a flexible sealing lip (36) on the underside thereof that is engageable by the upper end of said vial (11) when said closure member (15) is in said second position.

18. A test container according to claim 1, in which said vial (11) is shaped to contain said aqueous medium within a section (86) of said vial that is sealed from said test strip by a dislodgable stopper (90), the means for permitting contact of the test strip (14) with the medium (16) comprising means (35y) for dislodging said stopper (90) in response to movement of said closure member (15) to said second position.

19. A method of testing the effectiveness of sterilization comprising the steps of: placing with items to be sterilized a vial (11) containing a test strip (14) with a predetermined form of micro-organisms thereon and an aqueous growth medium (16) such that when brought in contact with viable micro-organisms on said test strip and incubated for a predetermined period will result in the evolution of detectable gas, maintaining the test strip (14) in said vial (11) out of contact with the said medium therein while exposing the test strip to the sterilizing atmosphere during a sterilization cycle along with the items to be sterilized, contacting the test strip with the medium in said vial following the sterilization cycle, sealing the medium and test strip in the vial, incubating the vial and its sealed contents for a predetermined period to cause any viable micro-organisms remaining following the sterilization cycle to evolve a gas, and analyzing the gas evolved in said sealed vial to determine the effectiveness of the sterilization cycle.

20. A method according to claim 19, wherein the said medium (16) includes a radio-active isotope component such that the gas evolved during incubation is radio-active, and including analyzing the radio-activity of said gas evolved in said sealed vial (11) to determine quantitatively the degree of sterilization effected.

21. A method according to claim 19 or 20, including fracturing said ampoule and sealing said vial substantially simultaneously.

22. A method according to claim 19, 20 or 21, including maintaining said test strip (14) out of contact with said aqueous medium (16) during a sterlizing cycle by containing said medium in a sealed frangible ampoule (12) disposed within said vial (11), and fracturing said ampoule (12) to contact said test strip with said medium (16) following exposure of  said
test strip to a sterilizing cycle.

_FIG. 1._

_FIG. 2._

0152298

2/5

Fig. 3.

Fig. 4.

Fig. 5.

Fig. 6.

Fig. 7.

fig. 8.

fig. 9.

0152298

Fig. 10.

Fig. 11.

Fig. 12.

Fig. 13.

Fig. 14.

Fig. 15.